# EUROPEAN PATENT SPECIFICATION

(11) **EP 3 586 752 B1**
(45) Date of publication and mention of the grant of the patent: **25.06.2025**
(21) Application number: 18757948.7
(22) Date of filing: 27.02.2018
(51) Int. Cl.: A61B 6/03, A61B 6/00

(54) **STATIC REAL-TIME CT IMAGING SYSTEM ADAPTABLE TO LARGE VISUAL FIELD REQUIREMENTS AND IMAGING METHOD**
AN ANFORDERUNGEN EINES GROSSEN SICHTFELDES ANPASSBARES STATISCHES ECHTZEIT-CT-BILDGEBUNGSSYSTEM UND BILDGEBUNGSVERFAHREN
SYSTÈME D'IMAGERIE DE TOMODENSITOMÉTRIE EN TEMPS RÉEL STATIQUE ADAPTABLE À DES EXIGENCES DE CHAMP VISUEL ÉTENDU ET PROCÉDÉ D'IMAGERIE

(30) Priority: 27.02.2017 CN 201710109426
(43) Date of publication of application: 01.01.2020
(73) Proprietor: Nanovision Technology (Beijing) Co., Ltd., Beijing 100094 (CN)
(72) Inventor: CAO, Hongguang, Beijing 100094 (CN); LI, Yunxiang, Beijing 100094 (CN); XING, Jinhui, Beijing 100094 (CN)
(74) Representative: Richards, John
(86) International application number: PCT/CN2018/077451
(87) International publication number: WO 2018/153382

(56) References cited:
- EP-A1- 0 543 626
- WO-A1-2016/029845
- CN-A- 101 801 272
- CN-A- 105 361 900
- CN-A- 106 353 350
- CN-B- 102 988 076
- JP-A- 2004 357 724
- US-A1- 2007 003 004
- US-A1- 2014 270 054
- US-B2- 8 699 657
- ROBB R A ET AL: "THE DSR: A HIGH-SPEED THREE-DIMENSIONAL X-RAY COMPUTED TOMOGRAPHY SYSTEM FOR DYNAMIC SPATIAL RECONSTRUCTION OF THE HEART AND CIRCULATION", IEEE TRANSACTIONS ON NUCLEAR SCIENCE, IEEE SERVICE CENTER, NEW YORK, NY, US, vol. NS-26, no. 2, 1 April 1979 (1979-04-01), pages 2713 - 2717, XP001377716, ISSN: 0018-9499

## Description

### BACKGROUND

### Technical Field

The invention relates to a static real-time CT imaging system adaptable to large visual field requirements, and also relates to a method for realizing real-time imaging based on the CT imaging system, belonging to the technical field of medical imaging.

### Related Art

CT is an abbreviation of Computed Tomography. The imaging principle of CT is as follows: X-ray beams and X-ray detectors with very high sensitivity are used to scan the cross section of a certain part of a human body layer by layer. A scintillating material on the X-ray detectors receives X-ray passing through this layer, the X-ray is converted into visible light, then the visible light is converted into an electrical signal by a photoelectric converter, the electrical signal is amplified, then converted into a digital signal through analog-digital conversion, and finally the digital signal is input into a computer for processing. In the computer, the selected layer is divided into a plurality of cubes with the same volume, which are called as voxels. After the information obtained by layer-by-layer cross-sectional scanning is calculated, an X-ray attenuation or absorption coefficient of each voxel is obtained, and then a matrix, i.e., a voxel digital matrix, is obtained through arrangement. The digital information in the voxel digital matrix is converted into small square blocks with unequal gray levels from black to white, which are called as pixels in the sense of 2D projection. A CT image is formed through arrangement according to the layer dividing mode.

The applicant has disclosed a static real-time CT imaging system in the Chinese patent application No. 201410425061.2. The system comprises an annular photon counting detector, an annular scanning X-ray source and a scanning sequence controller, wherein under the control of the scanning sequence controller, the annular scanning X-ray source emits a narrow beam of X-ray, which passes through the object to be measured and arrives at the corresponding annular photon counting detector. The annular photon counting detector sends the corresponding exposure information to a data acquisition processing unit and a human-computer interaction unit through a scanning host and a main control unit, and image reconstruction is completed in the data acquisition processing unit and the human-computer interaction unit. In the invention, by changing the X-ray projection position in turn through electronic control, the scanning speed is increased by tens of times, and dynamic 3D images can be obtained; by adopting the photon counting detector, absorption data and energy data can be obtained, and thus real-time data reconstruction is realized; by adopting the narrow beam of X-ray, high-quality images can be obtained at one-tenth dose of the traditional CT imaging system, and the patient is prevented from being excessively radiated.

On the other hand, cone-beam CT is a new stage in the development of CT. The cone-beam CT uses a flat panel detector to perform a series of radiation projection measurement from different angles to the object to be measured, and then obtains isotropic 3D structure information through a 3D reconstruction algorithm. In cone-beam CT detection, because of the limitation of the cone angle of the X-ray source and the size of the flat panel detector, it is often encountered that the size of the object to be measured exceeds the imaging field of view (FOV) of the cone-beam CT. In this case, the whole object to be measured cannot be scanned or reconstructed well by adopting conventional scanning methods and reconstruction algorithms. If the method of multi-time block-by-block scanning and reconstruction is adopted, the projection data corresponding to each block will be truncated vertically or horizontally. However, for the common CT reconstruction algorithm, if it is required that the object to be measured is completely covered by X-ray, the reconstruction effect is not ideal in the case of projection truncation.

In order to meet the needs of large visual field, the Chinese invention patent No. ZL 200910091282.X has discloses a detector-biased large-field-of-view cone-beam X-ray oblique scanning 3D digital imaging method. In this method, a planar array detector is biased, and a cone-beam X-ray generated by an X-ray source obliquely transilluminates the imaging area of the component at a certain angle relative to the length and width surface of the component. In the scanning process, the ray source and the planar array detector are stationary, the component rotates for 360 degrees at equal angular step around the rotating axis, and the planar array detector obtains the ray signal modulated by the component at each rotating angle. With the same system hardware and scanning speed, the invention can double the oblique scanning imaging field of view. The patent publication JP2004357724 A discloses a prior art system and method wherein the views between adjacent focal spots are interpolated by swinging the X-ray generation unit with an amplitude equal to the angle that separates the two focal spots.

### SUMMARY

In view of the shortcomings of the prior art, the primary technical problem to be solved by the invention is to provide a static real-time CT imaging system adaptable to large visual field requirements.

Another technical problem to be solved by the invention is to provide a method for realizing real-time imaging based on the CT imaging system.

The invention is defined by the enclosed claims.

Compared with the prior art, the static real-time CT imaging system and the imaging method, by emitting wide beam of X-rays through the multifocal annular X-ray sources, in cooperation with the non-inverse geometry imaging mode between the X-ray source and detector, can adapt to the large visual field requirements (i.e., FOV reaches about 450-500).

### BRIEF DESCRIPTION OF THE DRAWINGS

Fig. 1 is a mechanical structural schematic view of a static real-time CT imaging system;
Fig. 2 and Fig. 3 are mechanical structural schematic views of a scanning frame unit 2 in a static real-time CT imaging system;
Fig. 4 is a front view of a core part of a static real-time CT imaging system adaptable to large visual field requirements;
Fig. 5 is a side view of a core part of a static real-time CT imaging system adaptable to large visual field requirements;
Fig. 6 is a schematic view that a multifocal annular X-ray source emits X-rays sequentially and at interval;
Fig. 7 is a schematic view that a multifocal annular X-ray source performs parallel scanning;
Fig. 8 is a schematic view that a multifocal annular X-ray source performs interpolation scanning;
Fig. 9 is a schematic view that a multifocal annular X-ray source realizes oscillating interpolation;
Fig. 10 is a structural schematic view of an arc-shaped multifocal stationary anode grid-control ray source;
Fig. 11 is a schematic view of connection between stationary anode reflected ray tubes and a ray tube support in Fig. 10;
Fig. 12A is a front view of a connecting structure between stationary anode reflected ray tubes and grid control switches;
Fig. 12B is a side view of a connecting structure between stationary anode reflected ray tubes and grid control switches;
Fig. 13 is a schematic view of an entire ring structure consisting of a plurality of arc-shaped multifocal stationary anode grid-control ray sources;
Fig. 14 is a schematic view that multifocal annular X-ray sources perform circumferential multi-energy spectrum scanning;
Fig. 15 is a flowchart that a static real-time CT imaging system realizes image reconstruction.

### DETAILED DESCRIPTION

The technical content of the invention will be further described below in detail with reference to the drawings in combination with the specific embodiments.

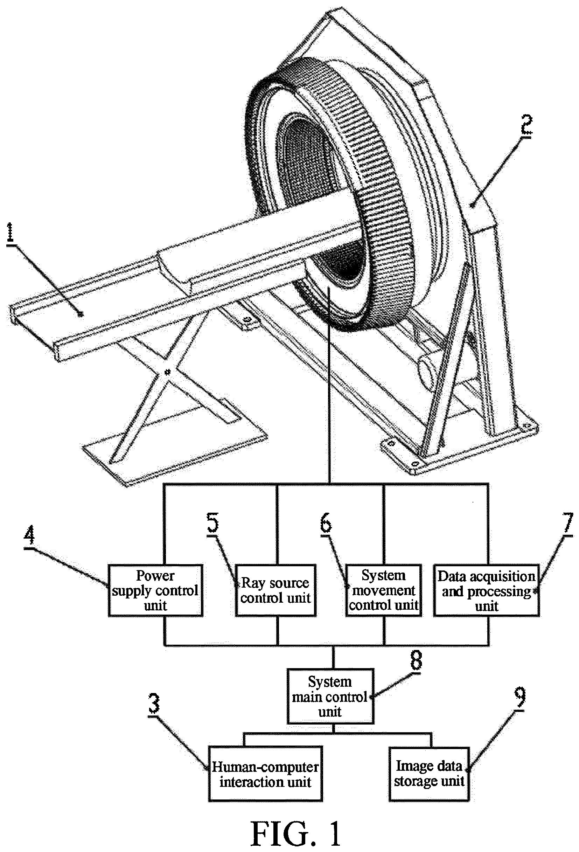

Fig. 1 is a mechanical structural schematic view of a static real-time CT imaging system. The static real-time CT imaging system specifically comprises a scanning bed unit 1, a scanning frame unit 2, a human-computer interaction unit 3, a power supply control unit 4, a ray source control unit 5, a movement control unit 6, a data acquisition processing unit 7, a system main control unit 8 and an image data storage unit 9, wherein the human-computer interaction unit 3, the power supply control unit 4, the ray source control unit 5, the movement control unit 6, the data acquisition processing unit 7, the system main control unit 8 and the image data storage unit 9 may be provided inside an annular CT scanning component, the annular CT scanning component is mounted on the scanning frame unit 2, and the scanning bed unit 1 passes through a circular space in the middle of the scanning frame unit 2.

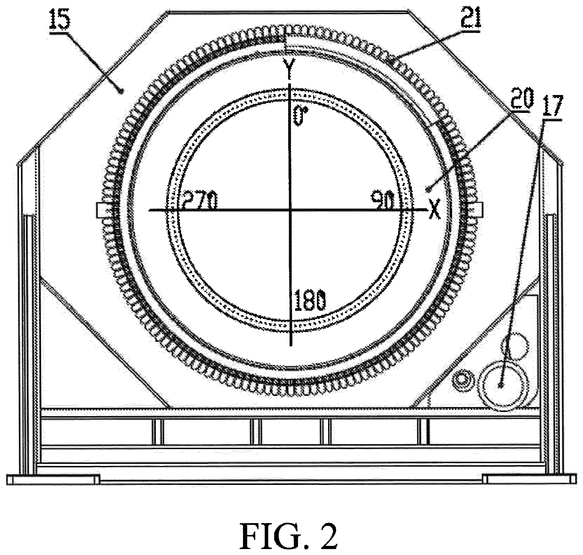

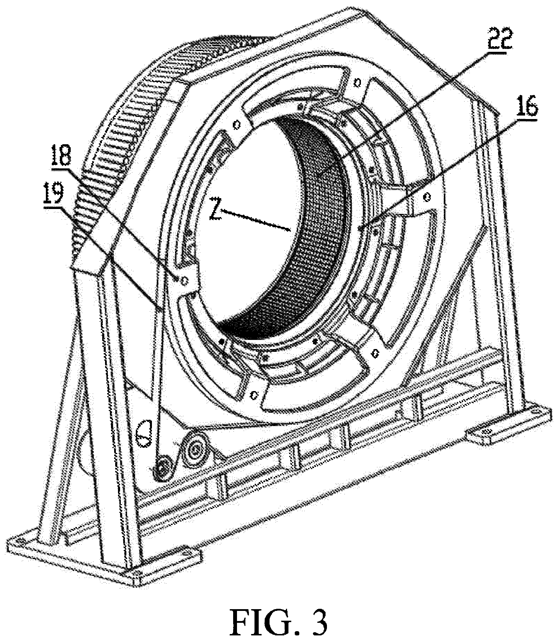

In one embodiment of the invention, the human-computer interaction unit 3, the power supply control unit 4, the ray source control unit 5, the movement control unit 6, the data acquisition processing unit 7 and the image data storage unit 9 are respectively connected with the system main control unit 8 to obtain operation instructions. Fig. 2 and Fig. 3 are mechanical structural schematic views of the scanning frame unit 2 in the static real-time CT imaging system. The scanning frame unit 2 further comprises a support 15, a main bearing 16, a driving motor 17, a belt pulley 18, a belt 19, a rotating support 20, a multifocal annular X-ray tube 21 and an annular photon detector 22. It should be noted that, in the imaging process of the static real-time CT imaging system, the rotating support 20 does not need to perform rotation imaging like ordinary CT, and the rotating support 20 is relatively stationary for most of the time. When necessary, the driving motor 17 can drive the belt pulley 18, the belt 19 and so on, such that the multifocal annular X-ray tube 21 on the rotating support 20 oscillates at a small angle along the circumference direction. This is to perform angle interpolation for fine reconstruction, which is not essentially a rotation imaging method like ordinary CT.

The static real-time CT imaging system, by emitting wide beam of X-rays through the multifocal annular X-ray sources, in cooperation with the non-inverse geometry imaging mode between the X-ray source and the detector, can adapt to the large visual field requirements (i.e., FOV reaches about 450-500). This will be described below in detail.

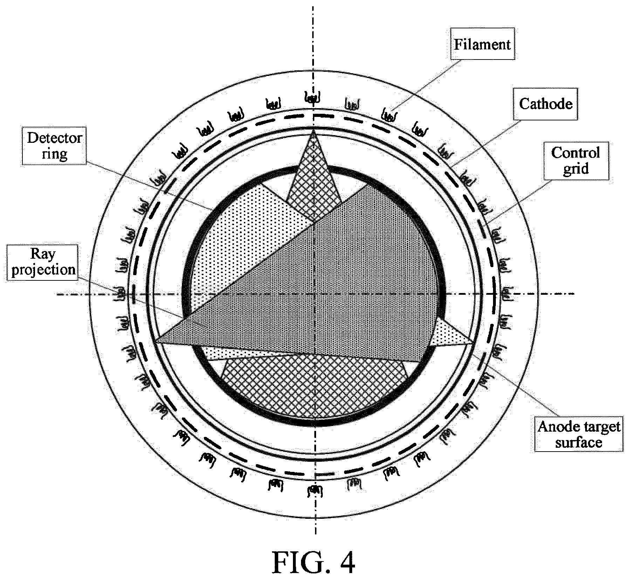

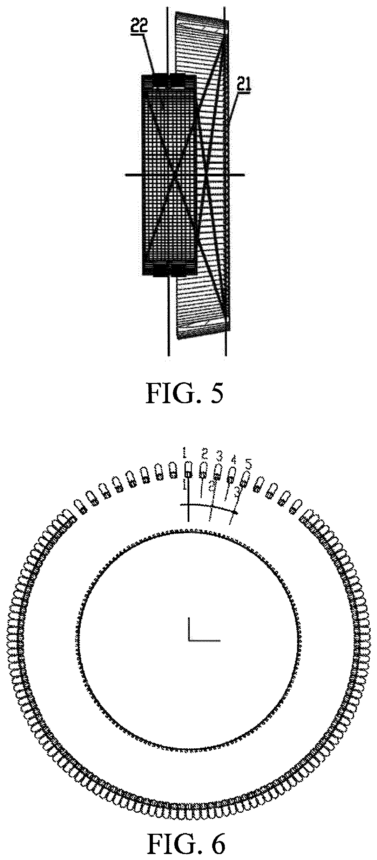

The static real-time CT imaging system adaptable to large visual field requirements is composed of a multifocal annular X-ray source and an annular photon detector, wherein the multifocal annular X-ray source 21 and the annular photon detector 22 are mounted on the rotating support 20, both of the multifocal annular X-ray source 21 and the annular photon detector 22 are on the same axis, and the axis is the Z axis commonly known in the field of CT. As illustrated in Fig. 4, the multifocal annular X-ray source emits a wide beam of X-ray, and the corresponding annular photon detector operates in overlapping manner. For example, the No. 1 scanning X-ray source operates corresponding to the No. 1, 2, 3, 4 and 5 photon detectors, the No. 2 scanning X-ray source operates corresponding to the No. 2, 3, 4, 5 and 6 photon detectors, and the No. 3 scanning X-ray source operates corresponding to the No. 3, 4, 5, 6 and 7 photon detectors, and so on. The corresponding operating mode is entire ring readout, that is, projection areas corresponding to a plurality of focuses are read out at one time. The focal plane of the multifocal annular X-ray source and the Z-direction central plane of the annular photon detector may overlap or not overlap. Considering from the angle of engineering implementation, it is easier to realize in engineering when the focal plane and the Z-direction central plane do not overlap. As illustrated in Fig. 5, when the focal plane and the Z-direction central plane do not overlap, the X-rays are obliquely emitted to the surface of the detector (i.e., the detector modules in the annular photon detector are not perpendicular to the corresponding incident X-rays). Necessary geometric correction is required during imaging. Specific geometric correction algorithm is a common technical means commonly known by one skilled in the art, which will be not repetitively described here.

The multifocal annular X-ray source may consist of a plurality of independent scanning X-ray sources closely and uniformly arranged in an annular shape, may consist of annular X-ray sources with a plurality of focuses on which a plurality of cathodes are uniformly distributed, or may be composed of arc-shaped X-ray sources with a plurality of focuses on which a plurality of cathodes are uniformly distributed. The annular photon detector preferably is composed of a plurality of photon counting detector modules closely and uniformly arranged in an annular shape. A non-inverse geometry imaging method is used between the scanning X-ray source and the corresponding photon counting detector module. The number of the scanning X-ray sources arranged on the circumference may be consistent or inconsistent with the number of the photon counting detectors. As mentioned above, the photon counting detectors which form the annular photon detector may also be replaced by direct conversion, energy differentiation type or scintillator-based integration type X-ray detectors.

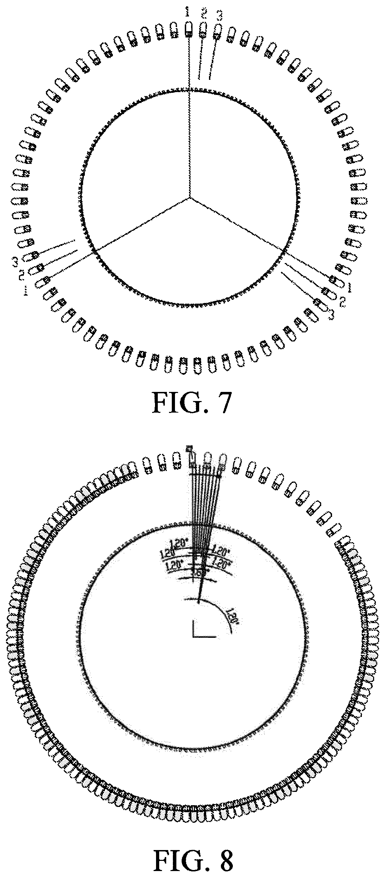

When the static real-time CT imaging system operates, the multifocal annular X-ray source is controlled by the ray source control unit and emits a wide beam of X-ray along the circumference direction, and the effect is equivalent to the circumferential rotation of the existing spiral CT. It should be noted that each scanning X-ray source of the multifocal annular X-ray source can emit X-rays clockwise or counter-clockwise. As illustrated in Fig. 6, each scanning X-ray source of the multifocal annular X-ray source can successively emit X-rays along the circumferential direction sequentially or at an interval of a plurality of scanning X-ray sources. As illustrated in Fig. 7, the multifocal annular X-ray source can emit X-rays simultaneously from one scanning X-ray source or simultaneously from a plurality of scanning X-ray sources in parallel. The maximum number of the scanning X-ray sources that emit X-rays in parallel is based on the premise that the X-rays emitted in parallel do not interfere with each other on the photon counting detectors, and preferably the circumferential distribution of the scanning X-ray sources that emit X-rays in parallel is circumferential uniform distribution.

The X-rays emitted by the multifocal annular X-ray source pass through the object to be measured and irradiate the corresponding photon counting detectors of the annular photon detector. The data acquisition processing unit is composed of a plurality of distributed subsystems, and embedded GPUs are integrated in the subsystems. The X-ray information received by the photon counting detectors is acquired and reconstructed by the data acquisition processing unit. The reconstructed image information is then transmitted to the image data storage unit and the human-computer interaction unit to complete image storage and visualization in the image data storage unit and the human-computer interaction unit. Of course, the existing CT data acquisition processing mode may be adopted, i.e., the data acquisition processing unit only acquires data, and then transmits the data to the image data storage unit for reconstruction and storage.

In the prior art, the number of focuses in the multifocal annular X-ray source determines the maximum circumferential projection density during static scanning. This projection density sometimes fails to meet some medical imaging needs which have higher requirements. In order to increase the maximum circumferential projection density, the static real-time CT imaging system provided by the invention can adopt the interpolation scanning mode. The circumferential projection density is enabled to be higher through the interpolation scanning mode, the energy spectrum scanning function can be realized and the multifocal parallel scanning speed is larger. As illustrated in Fig. 8, during interpolation scanning, the multifocal annular X-ray source rotates at a uniform speed for an angle not less than the included angle range between two adjacent focuses, and within the period of moving in the included angle range, the multifocal annular X-ray source completes a plurality of times of X-ray emission, it is equivalent to adding a plurality of focuses between two focuses, and the number of times of X-ray emission is equal to the number of times of interpolation scanning.

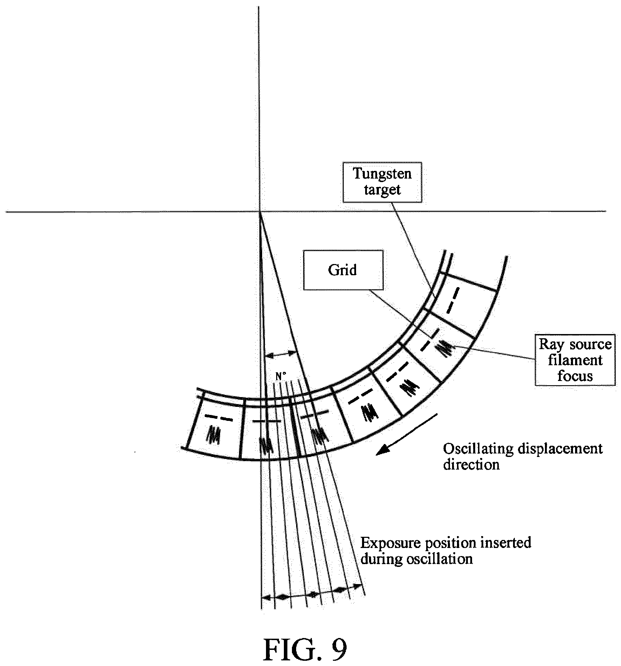

During interpolation scanning, only the multifocal annular X-ray source may oscillate at a small angle along the circumferential direction, or the multifocal annular X-ray source and the annular photon detector may oscillate relative to each other along the circumferential direction, or the rotating support does not move as a whole, and the scanning bed carries the human body to oscillate along the circumferential direction. Referring to the oscillating interpolation principle illustrated in Fig. 9, in one embodiment of the invention, if 150 focuses are uniformly distributed along the circumferential direction and the distance between adjacent focuses is 26 mm, the multifocal annular X-ray source and the annular photon detector oscillate for 2.4° (360/150=2.4) relative to each other, and the focuses increase the projection number through oscillating interpolation in the range of 26 mm, such that the projection number can be greatly increased and the quality of image reconstruction is effectively improved.

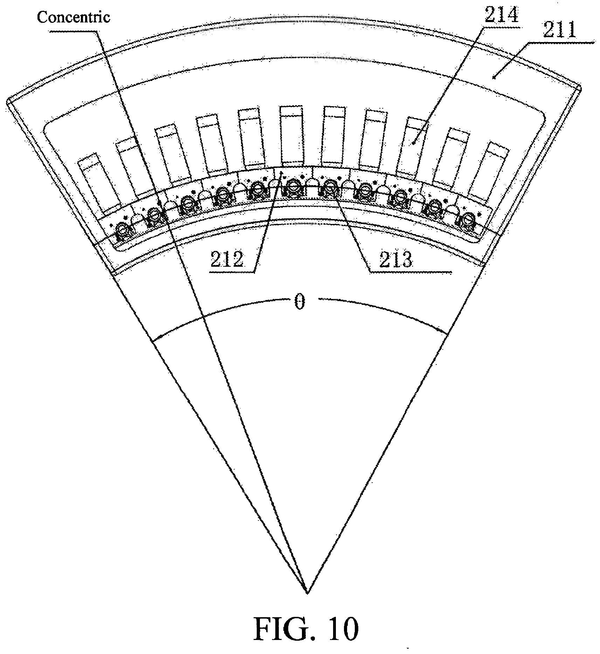

In the static real-time CT imaging system, the multifocal annular X-ray source can be illuminated alternately by adopting a grid-control reflecting target mode. This mode has the features of large power and high brightness, which is hundreds of times higher than the brightness of the deflected electron beam transmission target commonly used in the prior art. In order to realize the above mode, the multifocal annular X-ray source is preferably realized by splicing a plurality of arc-shaped multifocal stationary anode grid-control ray sources. The details are as follows:
As illustrated in Fig. 10, the arc-shaped multifocal stationary anode grid-control ray source comprises an arc-shaped ray source housing 211, a ray tube support 212, a plurality of stationary anode reflected ray tubes 213 and a plurality of grid control switches 214, wherein the plurality of stationary anode reflected ray tubes 213 are fixed on the arc-shaped ray source housing 211 through the ray tube support 212, and the focuses of the plurality of stationary anode reflected ray tubes 213 are distributed on the same distribution circle. More preferably, the focuses of the plurality of stationary anode reflected ray tubes 213 are uniformly distributed relative to the same distribution circle in a certain angle range α (360°≥α>0°); the plurality of grid control switches 214 and the plurality of stationary anode reflected ray tubes 213 are correspondingly connected to control the on/off of the plurality of stationary anode reflected ray tubes 213.

Taking the azimuth illustrated in Fig. 10 as an example, the specific structure of the arc-shaped multifocal stationary anode grid-control ray source will be described below.

As illustrated in Fig. 10, the arc-shaped ray source housing 211 is preferably a closed housing consisting of an inner arc plate, an outer arc plate, a left side plate, a right side plate, a front side plate and a rear side plate. The ray tube support 212, the plurality of stationary anode reflected ray tubes 213 and the plurality of grid control switches 214 are all provided inside the arc-shaped ray source housing 211. The included angle θ between the outer edge of the left side plate and the outer edge of the right side plate of the arc-shaped ray source housing 211 may be arbitrarily selected in the range of 0°-360°, and θ is preferably 360°/N, where N is a positive integer, e.g., θ is equal to 45°, 60°, 90°, 180°, etc. When θ=360°/N and N>1, N arc-shaped ray source housings 211 are spliced around the circumference, such that N arc-shaped multifocal stationary anode grid-control ray sources can form an entire ring structure, and the focuses of the plurality of stationary anode reflected ray tubes 213 can be distributed on the same distribution circle. It can be understood that, when θ=360°/N and N=1, the entire arc-shaped ray source housing is a circular ring structure, and the left side plate and the right side plate do not exist in theory.

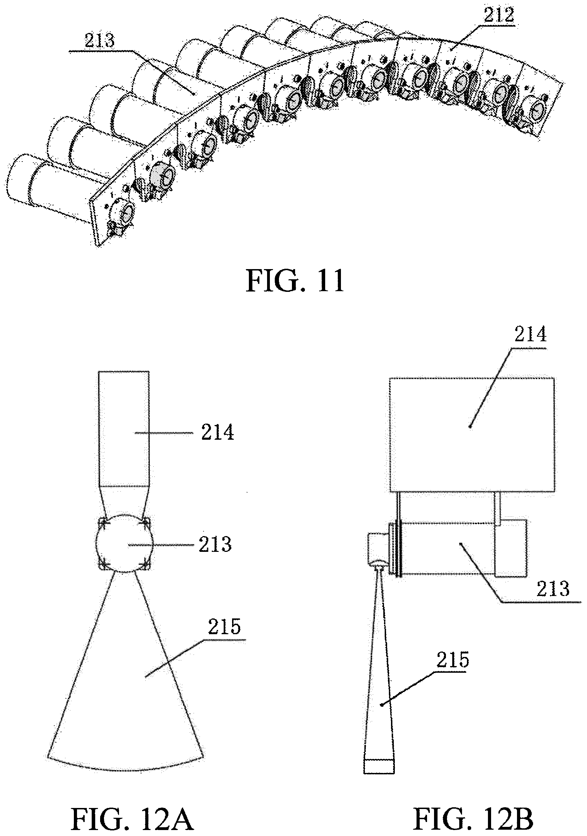

As illustrated in Fig. 11, the ray tube support 212 is preferably an arc-shaped support. The ray tube support 212 is fixed on the inner arc wall plate of the arc-shaped ray source housing 211 through bolts and other connectors, and the plurality of stationary anode reflected ray tubes 213 are fixed on the ray tube support 212.

A plurality of through holes are uniformly provided in the ray tube support 212, and the anode terminals of the plurality of stationary anode reflected ray tubes 213 respectively stretch out of the through holes in the ray tube support 212, and the plurality of stationary anode reflected ray tubes 213 are respectively fixed on the ray tube support 212 through flanges. In the actual structure, by finely adjusting the fixing positions and angles of the stationary anode reflected ray tubes 213 on the ray tube support 212, the focuses of the plurality of stationary anode reflected ray tubes 213 can be adjusted to the same circle. Hereinafter, the circle where the focuses of the plurality of stationary anode reflected ray tubes 213 are located is referred to as the distribution circle of the plurality of stationary anode reflected ray tubes 213.

The inner and outer end surfaces of the arc-shaped ray source housing 211 are arc surfaces, and the inner arc plate and the outer arc plate are respectively and concentrically provided with the distribution circle of the plurality of stationary anode reflected ray tubes 213. Of course, the inner arc plate and the outer arc plate may also be provided approximately concentrically. It is the most preferred that the inner arc plate and the outer arc plate are concentrically provided. The left and right end surfaces of the arc-shaped ray source housing 211 form an included angle θ and it is the most preferred that the extension lines of the left side plate and right side plate pass through the center of the distribution circle of a plurality of stationary anode emission ray tubes 3.

The focuses of the plurality of stationary anode reflected ray tubes 213 are uniformly distributed relative to the distribution circle in a certain angle range α, the angle range α is less than or equal to the angle θ between the out edge of the left side plate and the out edge of the right side plate of the arc-shaped ray source housing 211. In the actual structure, when the wall thickness of the left side plate and the right plate is small and the number of the stationary anode reflected ray tubes 213 provided in one arc-shaped ray source housing 211 is small, α and θ are approximately equal. More preferably, in the n stationary anode reflected ray tubes 213 provided in the same arc-shaped ray source housing 211, the angle between two adjacent stationary anode reflected ray tubes 213 is θ/n, and the angle between the leftmost and rightmost stationary anode reflected ray tubes 213 and the outer edge of the adjacent side plate is θ/2n. When the wall thickness of the left side plate and the right side plate is large or the number of the stationary anode reflected ray tubes 213 provided in one arc-shaped ray source housing 211 is large, α<θ, the angle between two adjacent stationary anode reflected ray tubes 213 is α/n, and the angle between the leftmost and rightmost stationary anode reflected ray tubes 213 and the outer edge of the adjacent side plate may be larger than α/2n or smaller than α/2n.

In the embodiment of the invention, the anode terminals of the used stationary anode reflected ray tubes 213 generate X-ray beams by using reflective stationary anode targets. The two ends of each stationary anode reflected ray tube 213 are an anode terminal and a cathode terminal respectively. A grid is provided near the cathode in the stationary anode reflected ray tube 213. As illustrated in Fig. 12A and Fig. 12B, each stationary anode reflected ray tubes 213 is provided with an independent grid control switch 214. The grid control switch 214 is fixed with the tube body of the stationary anode reflected ray tube 213 through a support, and the output end of the grid control switch 214 is connected to the grid of the stationary anode reflected ray tube 213 through a conducting wire to control the on/off of the stationary anode reflected ray tube 213 and realize the control of the ray output. The emitted X-ray beam 215 after being reflected by the anode terminal is as illustrated in Fig. 12A and Fig. 3B. Of course, the on/off of a plurality of adjacent stationary anode reflected ray tubes 213 may also be controlled by the same grid control switch 214. However, among the above control modes, it is preferred to adopt the control mode that the stationary anode reflected ray tubes 213 correspond to the grid control switches 214 one to one, as illustrated in the drawings.

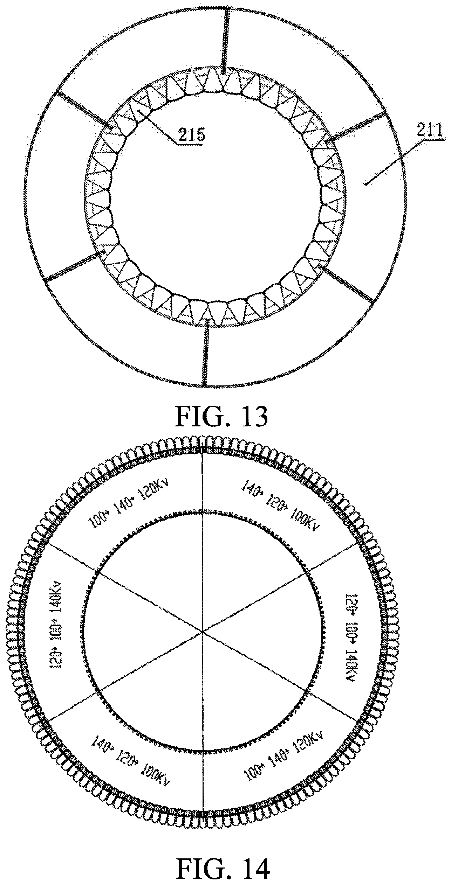

As illustrated in Fig. 13, by connecting the left side plates and the right side plates of N arc-shaped ray source housings 211 from head to tail, the N arc-shaped multifocal stationary anode grid-control ray sources can be spliced to form an "entire ring structure", such that the focuses of the plurality of stationary anode reflected ray tubes 213 can be distributed on the same distribution circle as uniformly as possible. By controlling the grid control switches 214 in the entire ring structure, the stationary anode reflected ray tubes 213 sequentially emit X-ray beams, such that sequential ray output for scanning in the direction of 360° can be realized. In the entire ring structure, the X-ray beam 215 emitted by each stationary anode reflected ray tube 213 illuminates the center of the entire ring.

When the angle between two adjacent stationary anode reflected ray tubes 213 provided in the same arc-shaped ray source housing 211 is θ/n, and the angle between the leftmost and rightmost stationary anode reflected ray tubes 213 and the outer edge of the adjacent side plate is θ/2n, by forming an entire ring structure through the N arc-shaped multifocal stationary anode grid-control ray sources, the focuses of all stationary anode reflected ray tubes 213 in the N arc-shaped multifocal stationary anode grid-control ray sources can be enabled to be uniformly distributed on the distribution circle. When the angle between two adjacent stationary anode reflected ray tubes 213 provided in the same arc-shaped ray source housing 211 is other values, in the entire ring structure consisting of N arc-shaped multifocal stationary anode grid-control ray sources, the focuses of all stationary anode reflected ray tubes 213 provided in N arc-shaped ray source housings 211 are distributed on the same distribution circle, and the focuses of the plurality of stationary anode reflected ray tubes 213 in each arc-shaped ray source housing 211 are uniformly distributed.

In the invention, a plurality of arc-shaped ray source housings may be spliced to form an entire ring structure, such that the focuses of all stationary anode reflected ray tubes in the plurality of arc-shaped multifocal stationary anode grid-control ray sources are uniformly distributed on the same distribution circle, so as to form a complete multifocal annular X-ray source. In the arc-shaped multifocal stationary anode grid-control ray sources, a plurality of grid control switches are correspondingly connected with a plurality of stationary anode reflected ray tubes, and the grid control switches can control the on/off of the circuit of the stationary anode reflected ray tubes, thus realizing the control of ray output. The arc-shaped multifocal stationary anode grid-control ray sources have the advantages that the structure is simple, the cost is low, rays with sufficient intensity can be generated, and a sufficient number of focuses are distributed in the circumferential direction.

As illustrated in Fig. 14, the static real-time CT imaging system can realize multi-mode energy spectrum scanning by using the multifocal annular X-ray source. The details are as follows:
Firstly, the multifocal annular X-ray source may perform energy spectrum scanning by using the instantaneous energy switching of a single scanning X-ray source, and can instantaneously switch to a variety of energy levels (such as switching between 100 kV, 120 kV and 140 kV illustrated in Fig. 14). The number of specifically switched energy levels is determined by the design requirement. After a certain scanning X-ray source performs energy spectrum scanning through instantaneous energy switching, a next scanning X-ray source under the control of scanning sequence performs energy spectrum scanning by adopting the same way until the entire scanning operation is completed. Secondly, the multifocal annular X-ray source may perform energy spectrum scanning by adopting circumferential intermittent energy switching. That is to say, all scanning X-ray sources of the multifocal annular X-ray source complete one circumferential scanning at the same energy level under the control of sequence, then are all switched to another energy level to repetitively complete the next circumferential scanning until switching to all energy levels is completed. Thirdly, the multifocal annular X-ray source may also be perform energy spectrum scanning by adopting a circumferential multi-energy spectrum scanning mode, i.e., the scanning X-ray sources distributed on the circumference are divided into a plurality of groups, each group is unified for one energy level, and after one circumferential scanning is completed under the control of sequence, the energy level of each group of scanning X-ray sources is switched to the corresponding next energy level, and the next circumferential scanning is repetitively completed until switching to all energy levels is completed.

It should be noted that the photon counting detectors in the invention may also be replaced by an integration type detectors to achieve the same function. The imaging effect of the integration type detectors is worse than that of the photon counting detectors, but the function is relatively stable due to the mature technology of the integration type detectors.

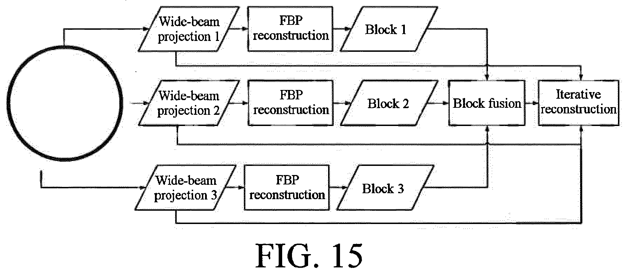

Fig. 15 is a flowchart that the static real-time CT imaging system provided by the invention realizes image reconstruction. In the static real-time CT imaging system, the data acquisition processing unit 7 is composed of a main control computer, a real-time reconstruction system and a visualization processor. The system main control unit 8 comprises a scanning sequence controller, high-speed data transmission channels, etc. The data processing function of the main control computer is accomplished by a main processor for parallel reconstruction of data and a storage device for storing voxel data. The scanning host comprises a plurality of photon counting detector modules 01-XX (XX is a positive integer, the same below). Each photon counting detector module comprises a plurality of photon counting detector units, e.g., the photon counting detector module 01 comprises photon counting detector units 01-XX.

In one preferred embodiment of the invention, each photon counting detector module is composed of 4*8=32 photon counting detector units, and each photon counting detector unit is composed of 32*32 photon counting detector pixels. The size of each pixel is 400*400 µm. The external size of each photon counting detector module is 51.2 mm*102.4 mm. The annular photon detector totally is composed of 50 photon counting detector modules. The diameter of the formed annular photon detector is about 800 mm and the width is 102.4 mm. There are a plurality of data acquisition channels, each of which corresponds to one or more photon counting detector modules. The data acquired by the plurality of photon counting detector modules 01-XX are transmitted to the data preprocessing module of the scanning host through the data acquisition channels 01-XX. The data preprocessing module is composed of a GPU, an ASIC or DSP, performs frame data preprocessing to original pixel exposure information transmitted by the data acquisition channels or the data subjected to data rearrangement and data correction, so as to form a frame data block, which is then transmitted to the parallel reconstruction module of the data acquisition processing unit through the high-speed data parallel transmission channels in the main control unit. The parallel reconstruction module may consist of a plurality of parallel GPUs or may consist of special ASIC. The reconstruction module reconstructs the uploaded frame or block data into block data. Since the above process is multi-channel parallel processing, the reconstruction speed can meet the requirement of real-time visual display.

An acquisition controller in the data acquisition processing unit 7 transmits commands to each photon counting detector module through an acquisition command channel under the control of scanning sequence controller to acquire pixel exposure information. Herein, the independent acquisition command channel is a multi-channel wide-digit bus, which ensures that all acquisition modules synchronously receive acquisition commands in parallel to ensure that the acquired frame data are data of the same frame period. The acquired pixel exposure information data are firstly integrated at the level of the photon counting detector units, then the data of the photon counting detector units are subjected to integration and process for the second time by the photon counting detector modules, including data splicing and module-level pixel data correction. Finally, the integrated or preliminarily reconstructed X-ray data are transmitted to a data preprocessor by the photon counting detector modules through parallel optical fiber data transmission channels.

The data preprocessor firstly performs frame data preprocessing to the X-ray data from different photon counting detector modules, including frame data rearrangement, frame data correction, frame data caching and real-time output of frame data. The real-time output frame data are transmitted to the real-time reconstruction system and the main control computer. Herein, the main task of the data preprocessor is to organize the data from the plurality of photon counting detector modules into complete data frames and data blocks, which are sent to the main control computer according to the format of frames and blocks. The data frame here refers to a data layer covering a complete range of 360°, and the data block refers to an array consisting of a plurality of data layers. The purpose of this data preprocessing mechanism is to complete the preliminary processing of some data processing tasks before the tasks are transmitted to the computer. The mechanism compiles the data acquired according to various acquisition orders edited by the controller into data formats of frames and blocks, and transfers the standard frame and block data to the main control computer for processing. Sometimes, we can put part of the preprocessing function of frame and block data in the data preprocessor, or put this part of data processing after the transmission to the main control computer, which is then completed by the main control computer. This part of preprocessing includes but not limited to error correction of single pixel, hardening correction, flat field correction of frame data, geometric correction of frame data or block data, time drift correction, energy correction, scattered ray suppression, etc.

In the prior art, multi-row spiral CT needs to rotate at high speed because of the X-ray source. In the process of rotation, it needs to transmit data to the computer by means of slip ring contact or wireless transmission. Compared with the existing multi-row spiral CT, the static real-time CT imaging system does not need a slip ring structure because of the absence of conventional rotation imaging links, and there is no mechanical movement false image. The static real-time CT imaging system can realize parallel data transmission by using optical fibers with better speed and reliability, the transmitted data flow increases, the reliability of data signals is improved, the overall structure is clearer and more reasonable, and the reliability and consistency of products are better. In this way, the real-time performance of the 3D reconstruction algorithm can be effectively guaranteed.

The parallel reconstruction module transmits the reconstructed voxel data to the visual image processor. Through a variety of mature visualization algorithms, it provides the data of various views to the observer, including but not limited to real-time projection 2D images, real-time 3D color rendered images, endoscope simulation images, surface rendered images, real-time multi-window gray absorption images, real-time energy palette images, and real-time DSA (Digital Subtraction Angiography) images. In one embodiment of the invention, the speed of providing visual images can reach 25 frames or blocks per second, and theoretically reconstruction speed of more than 1024 frames per second (i.e., 1024 fps) or blocks can be reached, which fully meets the need of human eyes for dynamic image observation and greatly improves the possibility of applying the static real-time CT imaging system to interventional therapy surgeries.

## Claims

1. A static real-time CT imaging system, comprising a scanning bed unit (1), a scanning frame unit (2), a human-computer interaction unit (3), a power supply control unit (4), a ray source control unit (5), a movement control unit (6), a data acquisition processing unit (7), a system main control unit (8) and an image data storage unit (9), wherein the scanning frame unit (2) comprises a multifocal annular X-ray source (21) and an annular photon detector (22);
the multifocal annular X-ray source (21) is composed of a plurality of scanning X-ray sources arranged in an annular shape, and the annular photon detector (22) is composed of a plurality of photon counting detector modules arranged in an annular shape;
each scanning X-ray source is configured to emit a wide beam of X-ray in turn which passes through the object to be measured and arrives at the corresponding photon counting detector module, and non-inverse geometry imaging is adopted between the scanning X-ray source and the corresponding photon counting detector module;
**characterized in that**
the multifocal annular X-ray source (21) is configured to rotate at a uniform speed for an angle larger than any included angle range separating two adjacent focuses, and within the period of moving in the included angle range, the multifocal annular X-ray source (21) is further configured to emit the wide beam of X-ray for many times to realize an interpolation scanning mode; and
the photon counting detector modules are configured to operate in an overlapping manner and to send the corresponding exposure information to the data acquisition processing unit (7), and the image is reconstructed in real time and visualized in the data acquisition processing unit (7).

2. The static real-time CT imaging system according to claim 1, wherein:
when the interpolation scanning mode is realized, only the multifocal annular X-ray source (21) oscillates at a small angle along a circumferential direction.

3. The static real-time CT imaging system according to claim 1, wherein:
in the multifocal annular X-ray source (21), a certain scanning X-ray source performs energy spectrum scanning in an instantaneous energy switching way, and a next scanning X-ray source under the control of scanning sequence performs energy spectrum scanning in the same way until the entire scanning operation is completed.

4. The static real-time CT imaging system according to claim 1, wherein:
in the multifocal annular X-ray source (21), each scanning X-ray source completes one circumferential scanning at the same energy level under the control of sequence, and then all scanning X-ray sources are switched to another energy level to repetitively complete next circumferential scanning until switching to all energy levels is completed.

5. The static real-time CT imaging system according to claim 1, wherein:
in the multifocal annular X-ray source (21), the scanning X-ray sources distributed on the circumference are divided into a plurality of groups, each group is unified for one energy level and completes one circumferential scanning under the control of sequence, and then each group of scanning X-ray sources is respectively switched to a corresponding next energy level to complete next circumferential scanning until switching to all energy levels is completed.

6. The static real-time CT imaging system according to claim 1, wherein:
the multifocal annular X-ray source (21) comprises a plurality of arc-shaped multifocal stationary anode grid-control ray sources, wherein the plurality of arc-shaped multifocal stationary anode grid-control ray sources are spliced to form an entire ring structure, and the focuses of all stationary anode reflected ray tubes (213) in the plurality of arc-shaped multifocal stationary anode grid-control ray sources are circumferentially distributed on the same distribution circle.

7. The static real-time CT imaging system according to claim 6, wherein:
the arc-shaped multifocal stationary anode grid-control ray source comprises an arc-shaped ray source housing (211), a ray tube support (212), a plurality of stationary anode reflected ray tubes (213) and a plurality of grid control switches (214), wherein the plurality of stationary anode reflected ray tubes (213) are fixed on the arc-shaped ray source housing (211) through the ray tube support (212), the focuses of the plurality of stationary anode reflected ray tubes (213) are uniformly distributed on the same distribution circle, and the plurality of grid control switches (214) and the plurality of stationary anode reflected ray tubes (213) are correspondingly connected.

8. The static real-time CT imaging system according to claim 7, wherein:
the ray tube support (212) is an arc-shaped support, the ray tube support (212) is fixed on the inner arc wall plate of the arc-shaped ray source housing (211), the plurality of stationary anode reflected ray tubes (213) are fixed on the ray tube support (212), and the focuses of the plurality of stationary anode reflected ray tubes (213) are uniformly distributed on the same distribution circle.

9. The static real-time CT imaging system according to claim 7, wherein:
a plurality of through holes are uniformly provided in the ray tube support (212), the anode terminals of the plurality of stationary anode reflected ray tubes (213) respectively stretch out of the through holes in the ray tube support (212), and the plurality of stationary anode reflected ray tubes (213) are respectively fixed on the ray tube support (212) through flanges.

10. The static real-time CT imaging system according to claim 7, wherein:
the inner arc wall plate and the outer arc wall plate of the arc-shaped ray source housing (211) are respectively and concentrically arranged with the distribution circles where the focuses of the plurality of stationary anode reflected ray tubes (213) are located; and
the extension lines of the left side plate and the right side plate of the arc-shaped ray source housing (211) pass through the centers of the distribution circles where the focuses of the plurality of stationary anode reflected ray tubes (213) are located.

11. A static real-time CT imaging control method, realized based on the static real-time CT imaging system according to any one of claims 1-10, wherein the static real-time CT imaging control method comprises the following steps:
controlling, by a scanning sequence controller, photon counting detector modules at different spatial locations and corresponding scanning X-ray sources to operate according to a predetermined scanning sequence; and
emitting, by the scanning X-ray sources, wide beam of X-rays in according to a predetermined emission sequence, and acquiring, by the corresponding photon counting detector modules, exposure information on the photon counting detector modules after the X-rays pass through the object to be measured in an overlapping manner,
**characterized by**
enabling the multifocal annular X-ray source (21) to rotate at a uniform speed for an angle larger than any included angle range separating two adjacent focuses, and within the period of moving in the included angle range, to emit, by the multifocal annular X-ray source (21), the wide beam of X-ray for many times to realize an interpolation scanning mode.

## Patentansprüche

1. Statisches Echtzeit-CT-Bildgebungssystem, das folgendes umfasst: eine Scannerbetteinheit (1), eine Scannerrahmeneinheit (2), eine Mensch-Computer-Interaktionseinheit (3), eine Stromversorgungssteuereinheit (4), eine Strahlungsquellensteuereinheit (5), eine Bewegungssteuereinheit (6), eine Datenerfassungsverarbeitungseinheit (7), eine Systemhauptsteuereinheit (8) und eine Bilddatenspeichereinheit (9), wobei die Scannerrahmeneinheit (2) eine multifokale ringförmige Röntgenstrahlenquelle (21) und einen ringförmigen Photonendetektor (22) umfasst;
wobei sich die multifokale ringförmige Röntgenstrahlenquelle (21) au seiner Mehrzahl scannender Röntgenstrahlenquellen zusammensetzt, die in einer Ringform angeordnet sind, und wobei sich der ringförmige Photonendetektor (22) aus seiner Mehrzahl von Photonen zählenden Detektormodulen zusammensetzt, die in einer Ringform angeordnet sind;
wobei jede Röntgenstrahlenquelle so gestaltet ist, dass sie einen breiten Röntgenstrahl emittiert, der wiederum durch das zu messende Objekt tritt und an dem entsprechende Photonen zählenden Detektormodul ankommt, und wobei nicht inverse geometrische Bildgebung zwischen der scannenden Röntgenstrahlenquelle und dem entsprechende Photonen zählenden Detektormodul angewendet wird;
**dadurch gekennzeichnet, dass**:
die multifokale ringförmige Röntgenstrahlenquelle (21) so gestaltet ist, dass sie sich für jeden Winkel, der größer ist als jeder eingeschlossene Winkel, der zwei benachbarte Brennpunkte trennt, mit gleichmäßiger Geschwindigkeit dreht, und wobei während des Zeitraums der Bewegung in dem Bereich des eingeschlossenen Winkels die multifokale ringförmige Röntgenstrahlenquelle (21) ferner so gestaltet ist, dass sie den breiten Röntgenstrahl viele Male emittiert, um einen Interpolationsscanmodus zu realisieren; und
die Photonen zählenden Detektormodule so gestaltet sind, dass sie auf überlappende Art und Weise arbeiten und dass sie die entsprechenden Expositionsinformationen an die Datenerfassungsverarbeitungseinheit (7) senden, und wobei das Bild in Echtzeit rekonstruiert und in der Datenerfassungsverarbeitungseinheit (7) visualisiert wird.

2. Statisches Echtzeit-CT-Bildgebungssystem nach Anspruch 1, wobei folgendes gilt:
wenn der Interpolationsscanmodus realisiert ist, oszilliert die multifokale ringförmige Röntgenstrahlenquelle (21) nur mit einem kleinen Winkel entlang einer umfänglichen Richtung.

3. Statisches Echtzeit-CT-Bildgebungssystem nach Anspruch 1, wobei folgendes gilt:
in der multifokalen ringförmigen Röntgenstrahlenquelle (21) führt eine bestimmte scannende Röntgenstrahlenquelle eine Energiespektrumabtastung durch sofortigen Energiewechsel aus und eine nächste scannende Röntgenstrahlenquelle führt gesteuert durch die Abtastsequenz eine Energiespektrumabtastung auf die gleiche Weise aus, bis der gesamte Abtastvorgang abgeschlossen ist.

4. Statisches Echtzeit-CT-Bildgebungssystem nach Anspruch 1, wobei folgendes gilt:
in der multifokalen ringförmigen Röntgenstrahlenquelle (21) führt jede scannende Röntgenstrahlenquelle eine umfängliche Abtastung auf dem gleichen Energieniveau gesteuert durch die Sequenz aus und danach werden alle scannenden Röntgenstrahlenquellen auf ein anderes Energieniveau umgeschaltet, um wiederholt die nächste umfängliche Abtastung vollständig auszuführen, bis alle Energieniveaus abgeschlossen sind.

5. Statisches Echtzeit-CT-Bildgebungssystem nach Anspruch 1, wobei folgendes gilt:
in der multifokalen ringförmigen Röntgenstrahlenquelle (21) sind die an dem Umfang verteilten scannenden Röntgenstrahlenquellen in eine Mehrzahl von Gruppen aufgeteilt, wobei jede Gruppe für ein Energieniveau vereint ist und eine umfängliche Abtastung gesteuert durch die Sequenz vollständig ausführt, und wobei danach jede Gruppe abtastender Röntgenstrahlenquellen entsprechend auf ein entsprechendes nächstes Energieniveau umgeschaltet wird, bis die Umschaltung auf alle Energieniveaus abgeschlossen ist.

6. Statisches Echtzeit-CT-Bildgebungssystem nach Anspruch 1, wobei folgendes gilt:
die multifokale ringförmige Röntgenstrahlenquelle (21) umfasst eine Mehrzahl bogenförmiger multifokaler, stationärer Anoden-Gittersteuerungs-Strahlenquellen, wobei die Mehrzahl bogenförmiger multifokaler, stationärer Anoden-Gittersteuerungs-Strahlenquellen gespleißt sind, so das seine ganze Ringstruktur gebildet wird, und wobei danach die Brennpunkte aller stationären Anoden-reflektierten Strahlenröhren (213) in der die Mehrzahl bogenförmiger multifokaler, stationärer Anoden-Gittersteuerungs-Strahlenquellen umfänglich auf dem gleichen Verteilungskreis verteilt werden.

7. Statisches Echtzeit-CT-Bildgebungssystem nach Anspruch 6, wobei folgendes gilt:
die bogenförmige multifokale, stationäre Anoden-Gittersteuerungs-Strahlenquelle umfasst ein bogenförmiges Strahlenquellengehäuse (211), einen Strahlenröhrenträger (212), eine Mehrzahl Anoden-reflektierter Strahlenröhren (213) und eine Mehrzahl von Gittersteuerschaltern (214), wobei die Mehrzahl Anoden-reflektierter Strahlenröhren (213) durch den Strahlenröhrenträger (212) an dem bogenförmigen Strahlenquellengehäuse (211) fixiert sind, wobei die Brennpunkte der Mehrzahl Anoden-reflektierter Strahlenröhren (213) gleichmäßig auf dem gleichen Verteilungskreis verteilt sind, und wobei die Mehrzahl von Gittersteuerschaltern (214) und die Mehrzahl von Anoden-reflektierten Strahlenröhren (213) entsprechend verbunden sind.

8. Statisches Echtzeit-CT-Bildgebungssystem nach Anspruch 7, wobei folgendes gilt:
der Strahlenröhrenträger (212) ein bogenförmiger Träger ist, wobei der Strahlenröhrenträger (212) an der innen Bogenwandplatte des bogenförmigen Strahlenquellengehäuses (211) fixiert ist, wobei die Mehrzahl Anoden-reflektierter Strahlenröhren (213) an dem Strahlenröhrenträger (212) fixiert sind, und wobei die Brennpunkte der Mehrzahl stationärer Anoden-reflektierter Strahlenröhren (213) gleichmäßig auf dem gleichen Verteilungskreis verteilt sind.

9. Statisches Echtzeit-CT-Bildgebungssystem nach Anspruch 7, wobei folgendes gilt:
eine Mehrzahl von Durchgangsöffnungen gleichmäßig in dem Strahlenröhrenträger (212) vorgesehen sind, wobei sich die Anodenanschlüsse der Mehrzahl stationärer Anoden-reflektierter Strahlenröhren (213) entsprechend aus den Durchgangsöffnungen in dem Strahlenröhrenträger (212) erstrecken, und wobei die Mehrzahl stationärer Anoden-reflektierter Strahlenröhren (213) durch Flansche entsprechend an dem Strahlenröhrenträger (212) fixiert sind.

10. Statisches Echtzeit-CT-Bildgebungssystem nach Anspruch 7, wobei folgendes gilt:
die innere Bogenwandplatte und die äußere Bogenwandplatte des bogenförmigen Strahlenquellengehäuses (211) sind entsprechend und konzentrisch in dem Verteilungskreis angeordnet, wo sich die Brennpunkte der Mehrzahl stationärer Anoden-reflektierter Strahlenröhren (213) befinden;
und die Erstreckungslinien der linksseitigen Platte und der rechtsseitigen Platte des bogenförmigen Strahlenquellengehäuses (211) verlaufen durch die Mittelpunkte der Verteilungskreise, wo sich die Brennpunkte der Mehrzahl stationärer Anoden-reflektierter Strahlenröhren (213) befinden.

11. Verfahren zur statischen Echtzeit-CT-Bildgebungssteuerung, realisiert auf der Basis des statischen Echtzeit-CT-Bildgebungssystems nach einem der Ansprüche 1 bis 10, wobei das Verfahren zur statischen Echtzeit-CT-Bildgebungssteuerung die folgenden Schritte umfasst:
durch eine Abtastsequenzsteuereinheit, Steuern der Photonen-zählenden Detektormodule an verschiedenen räumlichen Positionen und entsprechende abtastende Röntgenstrahlenquellen, um gemäß einer vorbestimmten Abtastsequenz zu arbeiten;
und, durch abtastende Röntgenstrahlenquellen, Emittieren von Röntgenstrahlen mit breitem Strahl gemäß einer vorbestimmten Emissionssequenz, und, durch die entsprechenden Photonen-zählenden Detektormodule, Erfassen von Expositionsinformationen an den Photonen-zählenden Detektormodulen nachdem die Röntgenstrahlen überlappend durch das zu messende Objekt getreten sind,
**gekennzeichnet durch**
Ermöglichen, dass sich die multifokale ringförmige Röntgenstrahlenquelle (21) für jeden Winkel, der größer ist als jeder eingeschlossene Winkel, der zwei benachbarte Brennpunkte trennt, mit gleichmäßiger Geschwindigkeit dreht sowie innerhalb des Zeitraums der Bewegung in dem Bereich des eingeschlossenen Winkels, um durch die multifunktionale ringförmige Röntgenstrahlenquelle (21) den breiten Röntgenstrahl vielfach zu emittieren, um einen Interpolationsscanmodus zu realisieren.

## Revendications

1. Système d'imagerie de tomodensitométrie en temps réel statique, comprenant une unité de lit de balayage (1), une unité de cadre de balayage (2), une unité d'interaction homme-machine (3), une unité de commande d'alimentation électrique (4), une unité de commande de source de rayons (5), une unité de commande de mouvement (6), une unité de traitement d'acquisition des données (7), une unité de commande principale de système (8) et une unité de stockage de données d'image (9), l'unité de cadre de balayage (2) comprenant une source de rayons X annulaire multifocale (21) et un détecteur de photons annulaire (22) ;
la source de rayons X annulaire multifocale (21) étant composée d'une pluralité de sources de rayons X à balayage agencées en forme d'anneau, et le détecteur de photons annulaire (22) étant composé d'une pluralité de modules détecteurs de comptage de photons en agencés en forme d'anneau ;
chaque source de rayons X à balayage étant conçue pour émettre à son tour un large faisceau de rayons X qui traverse l'objet à mesurer et arrive au module détecteur de comptage de photons correspondant, et l'imagerie à géométrie non inversée étant adoptée entre la source de rayons X à balayage et le module détecteur de comptage de photons correspondant ;
**caractérisé en ce que** la source de rayons X annulaire multifocale (21) est conçue pour tourner à une vitesse uniforme pour un angle plus grand que toute plage d'angles inclus séparant deux foyers adjacents, et
pendant la période de déplacement dans la plage d'angles inclus, la source de rayons X annulaire multifocale (21) est en outre conçue pour émettre le faisceau large de rayons X de nombreuses fois afin de réaliser un mode de balayage par interpolation ; et
les modules détecteurs de comptage de photons sont conçus pour fonctionner de manière chevauchante et pour envoyer les informations d'exposition correspondantes à l'unité de traitement d'acquisition de données (7), et l'image est reconstruite en temps réel et visualisée dans l'unité de traitement d'acquisition de données (7).

2. Système d'imagerie de tomodensitométrie en temps réel statique selon la revendication 1,
lorsque le mode de balayage par interpolation est réalisé, seule la source de rayons X annulaire multifocale (21) oscillant à un petit angle le long d'une direction circonférentielle.

3. Système d'imagerie de tomodensitométrie en temps réel statique selon la revendication 1,
dans la source de rayons X annulaire multifocale (21), une certaine source de rayons X à balayage effectuant un balayage du spectre d'énergie de manière à commutation d'énergie instantanée, et une source de rayons X à balayage suivante, sous le contrôle de la séquence de balayage, effectuant un balayage du spectre d'énergie de la même manière jusqu'à ce que l'ensemble de l'opération de balayage soit terminé.

4. Système d'imagerie de tomodensitométrie en temps réel statique selon la revendication 1,
dans la source de rayons X annulaire multifocale (21), chaque source de rayons X de balayage effectuant un balayage circonférentiel au même niveau d'énergie sous le contrôle de la séquence, puis toutes les sources de rayons X de balayage étant commutées à un autre niveau d'énergie pour effectuer de manière répétitive le balayage circonférentiel suivant jusqu'à ce que la commutation à tous les niveaux d'énergie soit terminée.

5. Système d'imagerie de tomodensitométrie en temps réel statique selon la revendication 1,
dans la source de rayons X annulaire multifocale (21), les sources de rayons X à balayage réparties sur la circonférence étant divisées en plusieurs groupes, chaque groupe étant unifié pour un niveau d'énergie et effectuant un balayage circonférentiel sous le contrôle de la séquence, puis chaque groupe de sources de rayons X à balayage étant respectivement commuté sur un niveau d'énergie suivant correspondant pour effectuer le balayage circonférentiel suivant jusqu'à ce que la commutation à tous les niveaux d'énergie soit terminée.

6. Système d'imagerie de tomodensitométrie en temps réel statique selon la revendication 1,
la source de rayons X annulaire multifocale (21) comprenant une pluralité de sources de rayons à commande par grille anode fixes multifocales en forme d'arc, la pluralité de sources de rayons à commande par grille anode fixes multifocales en forme d'arc étant épissées pour former une structure annulaire complète, et les foyers de tous les s à rayons réfléchis à anode fixe (213) dans la pluralité de sources de rayons à commande par grille anode fixes multifocales en forme d'arc étant distribués circonférentiellement sur le même cercle de distribution.

7. Système d'imagerie de tomodensitométrie en temps réel statique selon la revendication 6,
la source de rayons à commande par grille anode fixe multifocale en forme d'arc comprenant un boîtier de source de rayons en forme d'arc (211), un support de tube à rayons (212), une pluralité de tubes à rayons réfléchis à anode fixe (213) et une pluralité de commutateurs à commande par grille (214), la pluralité de tubes à rayons réfléchis à anode fixe (213) étant fixés sur le boîtier de source de rayons en forme d'arc (211) par l'intermédiaire du support de tube à rayons (212), les foyers de la pluralité de tubes à rayons réfléchis à anode fixe (213) étant uniformément répartis sur le même cercle de distribution, et la pluralité de commutateurs à commande par grille (214) et la pluralité de tubes à rayons réfléchis à anode fixe (213) étant connectés de manière correspondante.

8. Système d'imagerie de tomodensitométrie en temps réel statique selon la revendication 7,
le support de tube à rayons (212) étant un support en forme d'arc, le support de tube à rayons (212) étant fixé sur la plaque de paroi d'arc intérieure du boîtier de source de rayons en forme d'arc (211), la pluralité de tubes à rayons réfléchis à anode fixe (213) étant fixés sur le support de tube à rayons (212), et les foyers de la pluralité de tubes à rayons réfléchis à anode fixe (213) étant uniformément répartis sur le même cercle de distribution.

9. Système d'imagerie de tomodensitométrie en temps réel statique selon la revendication 7,
une pluralité de trous traversants étant uniformément ménagés dans le support de tube à rayons (212), les bornes d'anode de la pluralité de tubes à rayons réfléchis à anode fixe (213) s'étirant respectivement hors des trous traversants du support de tube à rayons (212), et la pluralité de tubes à rayons réfléchis à anode fixe (213) étant respectivement fixés sur le support de tube à rayons (212) par l'intermédiaire de brides.

10. Système d'imagerie de tomodensitométrie en temps réel statique selon la revendication 7,
la plaque de paroi d'arc intérieure et la plaque de paroi d'arc extérieure du boîtier de source de rayons en forme d'arc (211) étant respectivement et concentriquement disposées avec les cercles de distribution à l'endroit où sont situés les foyers de la pluralité de tubes à rayons réfléchis à anode fixe (213) ; et
les lignes d'extension de la plaque latérale gauche et de la plaque latérale droite du boîtier de source de rayons en forme d'arc (211) passant par les centres des cercles de distribution à l'endroit où sont situés les foyers de la pluralité de tubes à rayons réfléchis à anode fixe (213).

11. Procédé de commande d'imagerie de tomodensitométrie en temps réel statique, réalisé sur la base du système d'imagerie de tomodensitométrie en temps réel statique selon l'une quelconque des revendications 1 à 10, le procédé de commande d'imagerie de tomodensitométrie en temps réel statique comprenant les étapes suivantes consistant à :
commander, par un dispositif de commande de séquence de balayage, de modules détecteurs de comptage de photons à différents emplacements spatiaux et de sources de rayons X de balayage correspondantes afin qu'ils fonctionnent selon une séquence de balayage prédéfinie ; et
émettre, par les sources de rayons X à balayage, un large faisceau de rayons X selon une séquence d'émission prédéfinie, et acquérir, par les modules détecteurs de comptage de photons correspondants, des informations d'exposition sur les modules détecteurs de comptage de photons après que les rayons X ont traversé l'objet à mesurer d'une manière chevauchante,
**caractérisé par** les étapes consistant à
permettre à la source de rayons X annulaire multifocale (21) de tourner à une vitesse uniforme pour un angle plus grand que toute plage d'angles inclus séparant deux foyers adjacents, et dans la période de déplacement dans la plage d'angles inclus, émettre, par la source de rayons X annulaire multifocale (21), le faisceau large de rayons X de nombreuses fois pour réaliser un mode de balayage par interpolation.
